Europäisches Patentamt

(19) European Patent Office

Office européen des brevets

(11) EP 0 819 380 A1

(12) EUROPEAN PATENT APPLICATION

(43) Date of publication:
21.01.1998 Bulletin 1998/04

(51) Int. Cl.⁶: A01N 37/40, A61K 31/235

(21) Application number: 97300734.7

(22) Date of filing: 05.02.1997

(84) Designated Contracting States:
DE FR GB

(30) Priority: 07.06.1996 JP 145911/96

(71) Applicant: Hishida, Iwao
Toyonaka-shi (JP)

(72) Inventor: Hishida, Iwao
Toyonaka-shi (JP)

(74) Representative:
Mallalieu, Catherine Louise et al
D. Young & Co.,
21 New Fetter Lane
London EC4A 1DA (GB)

(54) **Bacteriocidal, antibacterial and bacteriostatic composition**

(57) Highly safe toxic p-oxybenzoic acid esters such as methyl, ethyl and butyl p-oxybenzoates possess a potent bacteriocidal, antibacterial or bacteriostatic activity against cariogenic Streptococcus mutans, Helicobacter pylori, Mycobacterium tuberculosis, influenza virus, HIV, herpes simplex and other bacteria and other viruses which lack any decisive remedy. The esters are extremely effective for the treatment or prevention of diseases caused by these bacteria and viruses.

EP 0 819 380 A1

**Description**

BACKGROUND OF THE INVENTION

Field of the Invention

The present invention relates to a bacteriocidal, antibacterial and bacteriostatic composition comprising a p-oxybenzoic acid ester as an active ingredient. More particularly, the present invention relates to a bacteriocidal, antibacterial and bacteriostatic composition comprising a p-oxybenzoic acid ester as an active ingredient, which exhibits a bacteriocidal, antibacterial or inactivating activity against bacteria or viruses, e.g., dental cariogenic Streptococcus mutans, Helicobacter pylori, Mycobacterium tuberculosis, influenza virus or herpes simplex.

Related Art Statement

Gingivitis and parodontitis are representative of periodontal diseases. One of causes for these diseases is attributed to cariogenic bacteria, an example of which is cariogenic Streptococcus mutans.

In recent years, gastric ulcer and duodenal ulcer are reportedly caused by Helicobacter pylori which is also one of bacteria. With increasing patients with such ulcers, a keen attention has been brought to development of bacteriocides against these bacteria.

Influenza is typically known as a disease caused by a virus but an effective drug for the treatment of influenza has not yet been developed.

A more serious disease would be HIV-induced AIDS causing death. It is one of the pressing needs of current times to develop drugs effective for AIDS.

On the other hand, p-oxybenzoic acid esters have been employed, for a long time, as a low toxic antiseptic and antimold agent for cosmetics, drugs, foodstuff, etc. and over a wide range of applications.

However, it was quite unknown whether p-oxybenzoic acid esters exhibit bacteriocidal, antibacterial or bacteriostatic activity against those bacteria or viruses described above, including cariogenic Streptococcus mutans, Helicobacter pylori, influenza virus, HIV, Mycobacterium tuberculosis and MRSA bacteria such as methicillin-resistant Staphylococcus aureus, etc.

p-Oxybenzoic acid esters are compounds widely known to be low-toxic and have no side effects to human. In this regard, it is established that p-oxybenzoic acid esters are highly safe drugs for administration. Another advantage is that p-oxybenzoic acid esters can be synthesized in a simple manner. If non-toxic p-oxybenzoic acid esters exerted a bacteriocidal, antibacterial or bacteriostatic activity against bacteria or viruses which were difficult to destroy, these compounds would be highly desirable as therapeutic agents for bacterial or viral diseases which have resisted all treatments to date.

The present inventor has made extensive studies on p-oxybenzoic acid esters focusing on their action against bacteria or viruses. It has been found that surprisingly p-oxybenzoic acid esters exhibit bacteriocidal, antibacterial and bacteriostatic activity which is extremely effective and destroys or inactivates cariogenic Streptococcus mutans, Helicobacter pylori, influenza virus, HIV, herpes simplex, Mycobacterium tuberculosis, etc which previously lacked any decisive remedy, and this would be favourable drugs for the treatment or prevention of these infections. It has also been found that by dissolving p-oxybenzoic acid esters in a solvent and preparing the solution into pharmaceutical preparations in a liquid, paste or jelly form, the bacteriocidal, antibacterial and bacteriostatic activity is exhibited more effectively.

SUMMARY OF THE INVENTION

According to one aspect of the present invention there is provided a bacteriocidal, antibacterial and bacteriostatic composition comprising as an active ingredient a p-oxybenzoic acid ester, which displays bacteriocidal, antibacterial, bacteriostatic or inactivating activity against bacteria or viruses including cariogenic Streptococcus mutans, Helicobacter pylori, Mycobacterium tuberculosis, influenza virus, HIV, herpes simplex, Pseudomonas aeruginosa, Staphylococcus aureus, Streptococcus pyogenes, Candida albicans, Escherichia coli and methicillin-resistant Staphylococcus aureus.

According to another aspect of the present invention there is provided use of p-oxybenzoic acid esters in a bacteriocidal, antibacterial and bacteriostatic composition for the treatment and/or prevention of bacterial or viral infections.

PREFERRED EMBODIMENTS OF THE INVENTION

Various preferred features and embodiments of the present invention will now be described by way of non-limiting example.

The composition of the present invention comprises a p-oxybenzoic acid ester as an active ingredient. Such a p-oxybenzoic acid ester includes a lower alkyl ester of p-oxybenzoic acid in which the alkyl moiety has 1 to 4 carbon atoms, such as methyl, ethyl, propyl and butyl esters; aralkyl esters such as benzyl ester, of p-oxybenzoic acid. These esters may also be used as an admixture of two or more.

Among the p-oxybenzoic acid esters, ethyl p-oxybenzoate is a commercially available compound that can be readily synthesized by well known procedures. The p-oxybenzoic acid esters such as methyl, ethyl, propyl and butyl p-oxybenzoates are also well known from their listings in the Existing Chemical Substance No. 3-1585, Japanese Pharmacopeia, Japanese Standards of Food Additives, Standards of Cosmetic Raw Materials, etc.

All of the esters are colorless crystalline or white crystalline powders and have the following physicochemical and toxicological properties:

Solubility in water at 25°C:

| methyl ester | 0.25 |
|---|---|
| ethyl ester | 0.17 |
| propyl ester | 0.05 |
| butyl ester | 0.02 |

Melting point:

| methyl ester | 125-128°C |
|---|---|
| ethyl ester | 116-118°C |
| propyl ester | 95-98°C |
| butyl ester | 69-72°C |

Acute toxicity in mice, p.o. (LD-50):

| methyl ester | 7800 mg/kg |
|---|---|
| ethyl ester | 5000 mg/kg |
| propyl ester | 8000 mg/kg |
| butyl ester | 17000 mg/kg |

Subacute toxicity in mice, p.o.:

These four (4) p-oxybenzoic acid esters were fed to mice, respectively, in a dose of 0.9 to 1.2 g/kg/day for 96 weeks (2 years) to examine subacute toxicity. Growth was not affected at all, indicating that toxicity of the p-oxybenzoic acid esters is extremely low in human and animal.

Since the p-oxybenzoic acid esters exhibit excellent bacteriocidal or antibacterial activity against cariogenic Streptococcus mutans (e.g., Streptococcus mutans IFO 13955, etc.), Helicobacter pylori and Mycobacterium tuberculosis, the compounds are extremely effective for the prevention and/or treatment of periodontal diseases caused by dental cariogenic bacteria such as cariogenic Streptococcus mutans; gastric or duodenal ulcer, tuberculosis, etc.

The p-oxybenzoic acid esters also possess a potent bacteriocidal, antibacterial or bacteriostatic action against an influenza virus (e.g., influenza virus type B, FluV-B, strain B/Lee/40; influenza virus type B, FluV-B, strain Yamagata/16/88), HIV (e.g., type HIV-1 (strain LA1), etc.), herpes simplex (e.g., HSV-1, strain VRIII, etc.). Therefore, the p-oxybenzoic acid esters are extremely effective for the prevention and/or treatment of diseases caused by infections with these viruses.

Furthermore, the p-oxybenzoic acid esters show a potent bacteriocidal or antibacterial activity against Pseudomonas aeruginosa, Staphylococcus aureus, Streptococcus pyogenes, Candida albicans, Escherichia coli and methi-

cillin-resistant <u>Staphylococcus</u> <u>aureus</u>. Therefore, the p-oxybenzoic acid esters are extremely effective for the prevention and/or treatment of diseases caused by infections with such bacteria or viruses as mentioned above.

The p-oxybenzoic acid esters may be administered orally or parenterally. The parenteral administration is typically by percutaneous, intramuscular, intravenous or transintestinal administration.

The pharmaceutical compositions can be prepared by blending the p-oxybenzoic acid esters as an active ingredient with pharmaceutically acceptable carriers conventionally used for pharmaceutical preparations, which include a solvent, a moisturizer, an excipient, a disintegrator, a binder, a lubricant, an antioxidant, a coating agent, a coloring agent, a corrigent, a surfactant, a plasticizer, etc. The compositions suitable for oral administration may be administered as conventional pharmaceutical preparations in the form of an elixir, granules, powders, capsules or tablets. Where the compositions are parenterally administered, the preparations generally take the form of an injection, a drip or a suppository.

To prepare the pharmaceutical compositions described above, a conventional method for preparing pharmaceutical preparations may be used, unless otherwise indicated.

Examples of suitable solvents include a monovalent alcohol such as methanol, ethanol or propanol; a polyvalent alcohol such as triethylene glycol, isopropylene glycol or propylene glycol; a polyalcohol such as polyethylene glycol or polypropylene glycol; and esters such as methyl lactate, ethyl lactate, butyl lactate, benzyl acetate, cyclohexyl acetate, methyl benzoate, propyl benzoate or butyl benzoate. Other conventional solvents such as dimethylsulfoxide may also be employed so long as the p-oxybenzoic acid esters are soluble and the solvent does not adversely affect the intended activity of the p-oxybenzoic acid esters.

Examples of suitable moisturizers include vegetable oils such as olive, orange, sesame, etc., beef tallow, paraffin or, a polyvalent alcohol such as vaseline.

The excipient may be one of those conventionally used in pharmaceutical compositions. Examples of such excipients include mannitol, xylitol, sorbitol, glucose, refined sugar, lactose, crystalline cellulose, sodium carboxymethyl cellulose, calcium hydrogenphosphate, wheat starch, rice starch, corn starch, potato starch, sodium carboxymethyl starch, dextrin, etc.

As the disintegrator there may be used any conventional compound generally employed in pharmaceutical compositions. Examples of the disintegrator include hydroxypropyl cellulose having a low degree of substitution, carboxymethyl cellulose, calcium carboxymethyl cellulose, or sodium carboxymethyl cellulose.

Examples of suitable binders include methyl cellulose, hydroxypropyl cellulose, hydroxypropylmethyl cellulose, polyvinylpyrrolidone, etc.

Examples of suitable lubricants include stearic acid, magnesium stearate, calcium stearate, etc.

Examples of suitable antacids include dibutyl hydroxytoluene (BHT), propyl gallate, etc.

Examples of suitable coating agents include hydroxypropylmethyl cellulose, hydroxypropyl cellulose, etc.

Examples of suitable colouring agents include tar pigment, titanium oxide, etc.

Examples of suitable corrigents include citric acid, adipic acid, ascorbic acid, menthol, etc.

Examples of suitable surfactants include polyoxyethylene-hardened castor oil, glycerine monostearate, sorbitan monostearate, sorbitan monopalmitate, sorbitan monolaurate, polyoxyethylene polyoxypropylene block copolymer, polysorbates, sodium lauryl sulfate, macrogols, sucrose fatty acid esters, etc.

Examples of suitable plasticizes include triethyl citrate, triacetin cetanol, etc.

The p-oxybenzoic acid esters may be prepared as pharmaceutical preparations in forms such as solutions, elixirs, tablets, injections and the like, preferably by using carriers and additives conventionally used for pharmaceutical preparations.

According to the present invention, preferred examples of the composition are pharmaceutical preparations in the form of a solution, an elixir, paste or jelly form, which may be obtained by dissolving the p-oxybenzoic acid esters in a solvent as described above.

More specifically, a solution obtained from p-oxybenzoic acid esters in a solvent mixture of ethanol or propylene glycol and water is preferably used as the pharmaceutical composition of the present invention. Alternatively, the p-oxybenzoic acid esters may be dissolved in polyethylene glycol or ethanol to form a solution and the resulting solution is another preferred pharmaceutical composition. The solution thus obtained may also be treated in a conventional manner to form pharmaceutical preparations in the form of paste or jelly. The paste or jelly preparations are also preferred in the present invention.

A solution of the p-oxybenzoic acid esters in the solvent, for example, as described above may also be administered as a mixture with a conventional or commercially available gargle, beverage, spray, tooth paste, etc. A solution of the p-oxybenzoic acid esters may also be incorporated in gum, throat candy, or crude drugs or other drugs.

A dose of the p-oxybenzoic acid ester may vary depending upon the condition of the subject and kind of disease but will generally be in the range of 100 to 200 mg daily for adults. The content of the p-oxybenzoic acid ester in the composition may also vary depending upon kind of preparation, disease of target, etc. but will generally be in the range of 50 to 200 mg.

The composition comprising the p-oxybenzoic acid ester as an active ingredient unexpectedly exhibits a potent bacteriocidal, antibacterial and bacteriostatic activity against bacteria and viruses such as <u>Streptococcus</u> <u>mutans</u>, <u>Helicobacter</u> <u>pylori</u>, <u>Mycobacterium</u> <u>tuberculosis</u>, influenza virus, herpes simplex, etc. Therefore, the composition of the present invention is extremely useful for the treatment and/or prevention of diseases caused by infections with these bacteria and viruses.

Hereinafter further preferred features and embodiments of the present invention will be described in more detail, with reference to the non-limiting examples below.

<u>Example 1</u>

<u>Bacteriocidal effect on Streptococcus mutans</u>

1) Preparation of pharmaceutical composition:

Solutions of p-oxybenzoic acid esters having the compositions given in Table 1 below were prepared.

Table 1

| Composition | | | | |
| --- | --- | --- | --- | --- |
| Composition | Active Ingredient | | Carrier | |
| | Compound | Amount (vol%) | Compound | Amount (vol%) |
| A | Propyl | | Ethanol | 10 |
| | p-oxybenzoate | 0.05 | Water | 90 |
| B | Ethyl | | Ethanol | 10 |
| | p-oxybenzoate | 0.05 | Water | 90 |
| C | Methyl | | Ethanol | 10 |
| | p-oxybenzoate | 0.05 | Water | 90 |
| D | Propyl | | Ethanol | 10 |
| | p-oxybenzoate | 0.025 | Water | 90 |
| | Ethyl | | | |
| | p-oxybenzoate | 0.025 | | |
| E | Propyl | | Ethanol | 10 |
| | p-oxybenzoate | 0.025 | Water | 90 |
| | Methyl | | | |
| | p-oxybenzoate | 0.025 | | |
| F | Propyl | | Ethanol | 10 |
| | p-oxybenzoate | 0.015 | Water | 90 |
| | Ethyl | | | |
| | p-oxybenzoate | 0.015 | | |
| | Methyl | | | |
| | p-oxybenzoate | 0.02 | | |
| G | Propyl | | Propylene | 10 |
| | p-oxybenzoate | 0.05 | glycol Water | 90 |
| H | Ethyl | | Propylene | 10 |
| | p-oxybenzoate | 0.05 | glycol Water | 90 |
| I | Methyl | | Propylene | 10 |
| | p-oxybenzoate | 0.05 | glycol Water | 90 |

2) Method:

Test strain <u>Streptococcus mutans</u> IFO 13955, which is cariogenic Streptococcus mutans, was incubated in agar medium (Eiken Kagaku K.K.) at 35°C for 18 to 24 hours. The cells were suspended in a sterilized phosphate buffer solution at about $10^8$ cells/ml.

1 ml of this cell suspension was added to and mixed with 10 ml of each of Compositions A through I shown in Table 1 above, each mixture was allowed to act at 20°C for a definite period of time. One platinum loop of the mixture was taken from each test tube and smeared on a post-incubation medium SCDLP (Nippon Pharmaceutical Co., Ltd) at time periods of 30 seconds, 1, 5, 10 and 30 minutes. After incubation at 35°C for 2 days, the test strain was observed to see whether it had grown or not, to determine whether the bacteria had survived or died.

For comparison, sterilized water was tested in a similar manner.

3) Results:

The results are shown in Table 2, wherein symbol + denotes that the bacteria were not dead and symbol - denotes that the bacteria were dead.

Table 2

| Test Results | | | | | | |
|---|---|---|---|---|---|---|
| Bacteria | Composition | Survival or Death of Bacteria | | | | |
| | | 30″ | 1′ | 5′ | 10′ | 30′ |
| Cariogenic Streptococcus mutans | A | + | + | - | - | - |
| | B | + | + | + | - | - |
| | C | + | + | + | + | - |
| | D | + | + | - | - | - |
| | E | + | + | - | - | - |
| | F | + | + | - | - | - |
| | G | + | + | - | - | - |
| | H | + | + | - | - | - |
| | I | + | + | - | - | - |
| | | + | + | - | - | - |
| | Control* | + | + | + | + | + |

\* Sterilized water was used.

The results shown in Table 2 reveal that the compositions comprising the p-oxybenzoic acid esters as an active ingredient exhibit a potent bacteriocidal activity against cariogenic <u>Streptococcus</u> <u>mutans</u>.

<u>Example 2</u>

<u>Bacteriocidal effect on Helicobacter pylori and Mycobacterium tuberculosis</u>

1) Preparation of pharmaceutical composition:

Compositions comprising p-oxybenzoic acid esters and having formulations shown in Table 3 were prepared in the form of solutions.

Table 3

| Composition of Pharmaceutical Preparations | | | | |
|---|---|---|---|---|
| Composition | Active Ingredient | | Carrier | |
| | Compound | Amount (vol%) | Compound | Amount (vol%) |
| A | Ethyl | | Propylene | 99 |
| | p-oxybenzoate | 1 | glycol | |
| B | Butyl | | Propylene | 99 |
| | p-oxybenzoate | 1 | glycol | |
| C | Methyl | | Propylene | 99 |
| | p-oxybenzoate | 1 | glycol | |
| D | Propyl | | Propylene | 99 |
| | p-oxybenzoate | 1 | glycol | |
| E | Ethyl | | | |
| | p-oxybenzoate | 1 | Ethanol | 99 |
| F | Butyl | | | |
| | p-oxybenzoate | 1 | Ethanol | 99 |
| G | Butyl | | | |
| | p-oxybenzoate | 1 | Ethanol | 99 |
| H | Propyl | | | |
| | p-oxybenzoate | 1 | Ethanol | 99 |

2) Method:

Three clinical strains of <u>Mycobacterium</u> <u>tuberculosis</u> and three clinical strains of <u>Helicobacter</u> <u>pylori</u> were used. After preincubation of the test strains, each medium was diluted with sterile physiological saline to about $10^8$ CFU/ml, which was provided for test as a bacterial dilution. Each of Compositions A through H shown in Table 3 was adjusted with sterile distilled water to a concentration of 0.5% and 1 ml of the bacterial solution was added to 9 ml of the thus prepared drug composition for test, followed by immediate mixing. Each mixture was allowed to stand for 30 minutes.

In the case of the clinical strains of <u>Helicobacter</u> <u>pylori</u>, after a definite time period allowed for action, one platinum loop of the mixture was smeared on Columbia sheep blood agar medium (BBL) followed by incubation at 35°C for 4 days under 10% $CO_2$. For control, the same procedures were performed with sterile physiological saline. Thereafter, it was observed to determine whether the bacteria grew or not.

In the case of the clinical strains of <u>Mycobacterium</u> <u>tuberculosis</u>, after a definite time period allowed for action, one platinum loop of the mixture was smeared on MYCOBACTERIA 7H11 AGAR (DIFCO) followed by aerobic incubation at 35°C for 3 weeks. For control, the same procedures were performed with sterile physiological saline. Thereafter, it was observed to determine whether the bacteria grew or not.

Where no bacteria grew after the incubation, it was judged that the composition had a bacteriocidal effect.

3) Results:

The results of the bacteriocidal effect on the clinical strains of <u>Helicobacter</u> <u>pylori</u> are shown in Table 4 below.

Table 4

| Test Results | | | |
|---|---|---|---|
| Composition | Helicobacter pylori | | |
| | Strain (1) | Strain (2) | Strain (3) |
| A | - | - | - |
| B | - | - | - |
| C | - | - | - |
| D | - | - | - |
| E | - | - | - |
| F | - | - | - |
| G | - | - | - |
| H | - | - | - |
| I | - | - | - |
| Control* | + | + | + |
| +: Growth of bacteria was noted.<br>-: No growth of bacteria was noted. | | | |

\* Physiological saline was used as control.

The results shown in Table 4 reveal that the compositions comprising the p-oxybenzoic acid esters as an active ingredient exhibit a potent bacteriocidal activity against Helicobacter pylori.

The results of the bacteriocidal effect on the clinical strains of Mycobacterium tuberculosis are shown in Table 5 below.

Table 5

| Test Results | | | |
|---|---|---|---|
| Composition | Mycobacterium tuberculosis | | |
| | Strain (1) | Strain (2) | Strain (3) |
| A | +* | +* | +* |
| B | - | - | - |
| C | +* | +* | +* |
| D | +* | - | - |
| E | - | - | - |
| F | - | - | - |
| G | - | - | - |
| H | - | - | - |
| I | - | - | - |
| Control* | + | + | + |
| +: Growth of bacteria was noted.<br>-: No growth of bacteria was noted.<br>+*:Growth of bacteria was noted but the amount decreased to less than 1/20 of control (physiological saline) | | | |

\* Physiological saline was used as control.

The results shown in Table 5 reveal that the compositions comprising the p-oxybenzoic acid esters as an active ingredient exhibit a potent bacteriocidal activity against <u>Mycobacterium</u> <u>tuberculosis</u>.

<u>Example 3</u>

<u>Inactivation of HIV</u>

1) Preparation of composition:

By mixing 1 % butyl p-oxybenzoate or 1 % methyl p-oxybenzoate, 1% dimethylsulfoxide and 98% water, Composition A containing butyl p-oxybenzoate and Composition B containing methyl p-oxybenzoate were prepared in the form of solutions.

2) Method:

Type HIV-1 (LAI strain) and MT-4 cells were employed for the test. Incubation was carried out in 10% FCS-supplemented RPMI 1640 at 37°C in a $CO_2$ incubator (5% $CO_2$).

Compositions A and B were diluted with water to make diluted compositions having the concentrations given in Table 6. Each of the thus obtained compositions was mixed with the viral solution in an equal amount followed by incubation at room temperature for 60 minutes. Thereafter $TCID_{50}/200$ μl was determined in MT-4 cells using a 96-well microplate so as to quantitatively determine the infectivity titer of the virus which survived. The infectivity titer was evaluated 5 days after inoculation with the virus. The cell count per well was adjusted to $1 \times 10^5$ cells/200 μl.

3) Results:

The results of the HIV inactivating effect of the compositions are shown in Table 6 below.

Table 6

| Results of HIV inactivation | | |
|---|---|---|
| Composition | Final Concentration of Active Ingredient (%) | Effect of Inactivation ($TCID_{50}/100$ μl) |
| A | 0.5 | 101.5 |
| | 0.05 | 102.17 |
| B | 0.5 | 102.32 |
| | 0.05 | 103.17 |
| Control* | - | 103.5 |

* Physiological saline was used for control.

As is clearly seen from the results of Table 6, ethyl p-oxybenzoate exhibits a potent inactivation effect against HIV in a concentration-dependent fashion.

<u>Example 4</u>

<u>Inactivation of herpes simplex I</u>

1) Preparation of composition:

By mixing 0.5% butyl p-oxybenzoate, 60.0% water, 20.0% ethanol and 20% propylene glycol, a composition comprising butyl p-oxybenzoate as an active ingredient was obtained in a solution form.

2) Method:

The composition prepared in 1) was mixed with the viral solution in a definite mixing ratio to maintain contact at room temperature, followed by 10-fold serial dilution from 1- to $10^7$-fold. The dilution was inoculated with culture cells.

After incubation for 5 days, the viral infectivity titer ($TCID_{50}/25$ µl) was determined using cytopathic effect (CPE) as an index to evaluate the effect of the composition against a virus.

As the virus, herpes simplex I (HSV-1, strain VRIII) was used and Vero cells were used as the culture cells.

The virus was inoculated with the culture cells in a culture flask of 75 $cm^2$ for adsorption at 37°C for an hour. Then 2% FCS-supplemented Eagle's MEM medium was added to the system followed by incubation at 37°C.

When CPE reached 75% or more, the culture supernatant and the infected cells were lyophilized and then thawed. After repeating the procedure 3 times, centrifugation was performed at 4°C for 20 minutes at 3000 rpm. The resulting supernatant was used as the viral solution.

The composition prepared in 1) and 70% ethanol for control were mixed with each viral solution in mixing ratios of 1:4, 1:9 and 1:99, respectively, and contact maintained for an hour at room temperature. The same procedure was repeated except using 2% FCS-supplemented Eagle's MEM medium in place of the composition. The resulting solution was used as a viral solution for control. Furthermore, the same evaluation was made using 2% FCS-supplemented Eagle's MEM medium in place of the viral solution, to confirm cytotoxicity.

The mixture of the composition and the viral solution in various mixing ratios was subjected to 10-fold serial dilution from 1- to $10^7$-fold using 2% FCS-supplemented Eagle's MEM medium. Each dilution series was inoculated with cells (4 wells each)(25 µl/well) which were cultured on a 96-well microplate. After HSV-1 was inoculated with Vero cells, the viral infectivity titer $TCID_{50}/20$ µl was determined. By comparing with the infectivity titer of the virus control determined at the same time, the effect of the composition on the virus was evaluated.

With respect to the mixing ratios judged to be effective in the above test, the mixtures were further contacted at room temperature for 0, 5, 15 and 30 minutes to evaluate the inactivation effect.

3) Results:

The composition obtained in 1) above and 70% ethanol were mixed with the HSV-1 viral solution in mixing ratios of 1:4, 1:9 and 1:99, respectively, and contact maintained for an hour at room temperature. The viral infectivity titer is shown in Table 7.

Table 7

| Viral infectivity titer when contacted for an hour ($TCID_{50}/25$ µl) | | | |
|---|---|---|---|
| Composition : Viral solution | Butyl p-oxybenzoate | 70% Ethanol | Virus Control[*1] |
| 1 : 4 | ≤$3.2 \times 10^1$ | $3.2 \times 10^4$ | $5.6 \times 10^4$ |
| 1 : 9 | ≤$3.2 \times 10^0$ | $5.6 \times 10^4$ | $3.2 \times 10^4$ |
| 1 : 99 | $1.8 \times 10^4$ | $1.0 \times 10^5$ | $3.2 \times 10^4$ |

*1 : For the virus control, 2% FCS-supplemented Eagle's MEM medium was used in place of the composition.

As is evident from the results shown in Table 7, when the mixing ratios of the composition to the viral solution were 1 : 4 and 1 : 9, the inactivation effect was noted as compared to the virus control.

Next, the mixing ratio of the composition to the viral solution was set as 1:9, as shown to be effective in the above test, and the contact time was shortened to confirm the inactivation effect.

The viral infectivity titer for various contact time periods is shown in Table 8.

Table 8

| Viral infectivity titer for various contact time periods (TCID$_{50}$/25 µl) | | | |
|---|---|---|---|
| Contact Time | Butyl p-oxybenzoate | 70% Ethanol | Virus Control[*1] |
| 0 minute | 5.6 x 10$^3$ | 3.2 x 10$^4$ | 1.8 x 10$^4$ |
| 5 minutes | 3.2 x 10$^0$ | 3.2 x 10$^4$ | 3.2 x 10$^4$ |
| 15 minutes | 3.2 x 10$^0$ | 1.8 x 10$^4$ | 1.8 x 10$^4$ |
| 30 minutes | 3.2 x 10$^0$ | 1.8 x 10$^3$ | 5.6 x 10$^4$ |
| 1 hour | 3.2 x 10$^0$ | 5.6 x 10$^4$ | 3.2 x 10$^4$ |

*1 : For the virus control, 2% FCS-supplemented Eagle's MEM medium was used in place of the composition.

As is evident from the results shown in Table 8, the virus was insufficiently inactivated immediately after contact of the composition with the viral solution (0 minute) but after contact for 5 minutes or longer, the inactivation effect was sufficiently established.

Example 5

Inactivation effect on influenza virus B

1) Preparation of Composition:

By mixing 0.5% butyl p-oxybenzoate, 60.0% water, 20.0% ethanol and 20% propylene glycol, a composition comprising butyl p-oxybenzoate as an active ingredient was obtained in a solution form.

2) Method:

The composition prepared in 1) was mixed with the viral solution in a definite mixing ratio to maintain contact at room temperature, followed by 10-fold serial dilution from 1- to 10$^7$-fold. The dilution was inoculated with culture cells.

After incubation for 5 days, the viral infectivity titer (TCID$_{50}$/25 µl) was determined using CPE as an index to evaluate the effect of the composition against a virus.

As the virus, influenza virus type B (FluV-B, strain B/Lee/40) herpes simplex I (HSV-1, strain VRIII) was used and MDCK cells were used as the culture cells.

The virus was inoculated with the culture cells in a culture flask of 75 cm$^2$ for adsorption at 37°C for an hour. Then medium for isolation of influenza virus was added to the system followed by incubation at 37°C.

When CPE reached 75% or more, the culture supernatant was centrifuged at 4°C for 20 minutes at 3000 rpm. The resulting supernatant was used as the viral solution.

The composition prepared in 1) and 70% ethanol for control were mixed with each viral solution in mixing ratios of 1 : 4, 1 : 9 and 1 : 99, respectively, and contact maintained for an hour at room temperature.

The same procedure was repeated except for using 2% FCS-supplemented Eagle's MEM medium in place of the composition. The resulting solution was used as a viral solution for control. Furthermore, the same evaluation was made using 2% FCS-supplemented Eagle's MEM medium in place of the viral solution, whereby cytotoxicity was confirmed.

The mixture of the composition and the viral solution in various mixing ratios was subjected to 10-fold serial dilution from 1- to 10$^7$-fold using 2% FCS-supplemented Eagle's MEM medium. Each dilution series was inoculated with cells (4 wells each) (25 µl /well) which were cultured on a 96-well microplate. After FluV-B was inoculated with MDCK cells, the viral infectivity titer TCID$_{50}$/20 µl was determined. By comparing with the infectivity titer of the virus control determined at the same time, the effect of the composition on the virus was evaluated.

With respect to the mixing ratios judged to be effective in the above test, the mixtures were further contacted at room temperature for 0, 5, 15 and 30 minutes to evaluate the inactivation effect.

3) Results:

The composition obtained in 1) above and 70% ethanol were mixed with the HSV-1 viral solution in mixing ratios of 1:4, 1:9 and 1:99, respectively, and contact maintained for an hour at room temperature. The viral infectivity titer is shown in Table 9.

Table 9

| Viral infectivity titer when contacted for an hour (TCID$_{50}$/25 µl) | | | |
|---|---|---|---|
| Composition : Viral solution | Butyl p-oxybenzoate | 70% Ethanol | Virus Control[*1] |
| 1 : 4 | ≤3.2 x 10$^0$ | 5.6 x 103 | 5.6 x 10$^3$ |
| 1 : 9 | ≤3.2 x 10$^0$ | 3.2 x 10$^3$ | 5.6 x 10$^3$ |
| 1 : 99 | 3.2 x 10$^3$ | 3.2 x 10$^3$ | 3.2 x 10$^3$ |

*1 : For the virus control, 2% FCS-supplemented Eagle's MEM medium was used in place of the composition.

As is evident from the results shown in Table 9, when the mixing ratios of the composition to the viral solution were 1 : 4 and 1 : 9, the inactivation effect was noted as compared to the virus control.

Next, the mixing ratio of the composition to the viral solution was set as 1:9, as shown to be effective in the above test, and the contact time was shortened to confirm the inactivation effect.

The viral infectivity titer in various contact time periods is shown in Table 10.

Table 10

| Viral infectivity titer for various contact time periods (TCID$_{50}$/25 µl) | | | |
|---|---|---|---|
| Contact Time | Butyl p-oxybenzoate | 70% Ethanol | Virus Control[*1] |
| 0 minute | 3.2 x 10$^0$ | 1.8 x 10$^3$ | 1.8 x 10$^3$ |
| 5 minutes | 3.2 x 10$^0$ | 1.8 x 10$^3$ | 1.8 x 10$^3$ |
| 15 minutes | 3.2 x 10$^0$ | 1.8 x 10$^3$ | 3.2 x 10$^3$ |
| 30 minutes | 3.2 x 10$^0$ | 3.2 x 10$^3$ | 3.2 x 10$^3$ |
| 1 hour | 3.2 x 10$^0$ | 5.6 x 10$^3$ | 5.6 x 10$^3$ |

*1 : For the virus control, 2% FCS-supplemented Eagle's MEM medium was used in place of the composition.

As is evident from the results shown in Table 10, the inactivation effect was noted immediately after the contact of the composition with the viral solution.

Example 6

Bacteriocidal effect on Pseudomonas aeruginosa, Staphylococcus aureus, Streptococcus pyogenes and Candida albicans

1) Preparation of pharmaceutical composition:

Compositions comprising p-oxybenzoic acid esters and having formulations shown in Table 11 were prepared in the form of solutions.

Table 11

| Composition of Pharmaceutical Preparation | | | | |
|---|---|---|---|---|
| Composition | Active Ingredient | | Carrier | |
| | Compound | Amount (vol%) | Compound | Amount (vol%) |
| A | Butyl p-oxybenzoate | 0.05 | Polyethylene glycol | 99.95 |
| B | Ethyl p-oxybenzoate | 0.05 | Polyethylene glycol | 99.95 |
| C | Methyl p-oxybenzoate | 0.05 | Polyethylene glycol | 99.95 |
| D | Propyl p-oxybenzoate | 0.05 | Polyethylene glycol | 99.95 |

2) Method:

As test strains, there were employed Pseudomonas aeruginosa IFO 13275, Staphylococcus aureus IFO 12732, Streptococcus pyogenes IID 712 and Candida albicans IFO 1594.

When Pseudomonas aeruginosa and Staphylococcus aureus were employed, each test strain was incubated in agar medium (Eiken Kagaku K.K.) at 35°C for 18 to 24 hours. The cells were suspended in a sterilized phosphate buffer solution at about $10^8$ cells/ml.

When Streptococcus pyogenes was employed, the test strain was incubated in brain heart infusion agar medium (Difco) at 35°C for 18 to 24 hours. The cells were suspended in a sterilized phosphate buffer solution at about $10^8$ cells/ml.

When Candida albicans was employed, the test strain was incubated in potato dextrose agar medium (Eiken Kagaku K.K.) at 25°C for 2 days. The cells were suspended in a sterilized phosphate buffer solution at about $10^7$ cells/ml.

1 ml of each of the cell suspensions was added to and mixed with 10 ml of each of Compositions A through D shown in Table 11 above, the mixture was allowed to act at 20°C for a definite period of time. One platinum loop of the mixture was taken from a test tube and smeared on a post-incubation medium at time periods of 5, 15 and 30 minutes. After incubation, the test strain was observed to see whether it grew or not, to determine whether the bacteria had survived or died. As a post-incubation medium for Pseudomonas aeruginosa, Staphylococcus aureus and Streptococcus pyogenes was used SCDLP (Nippon Pharmaceutical Co., Ltd.) and the post-incubation was performed at 35°C for 2 days. For Candida albicans, GPLP medium (Nippon Pharmaceutical Co., Ltd.) was used as the post-incubation medium and the incubation was performed at 25°C for 7 days.

For comparison, sterilized water was tested in a similar manner.

3) Results:

The results are shown in Table 12, wherein symbol + denotes that the bacteria were not dead and symbol - denotes that the bacteria were dead.

Table 12

| Test Results | | | | |
|---|---|---|---|---|
| Bacteria | Composition | Survival or Death of Bacteria | | |
| | | 5 mins | 15 mins | 30 mins |
| Pseudomonas aeruginosa | A | - | - | - |
| | B | - | - | - |
| | C | - | - | - |
| | D | - | - | - |
| | Control* | + | + | - |
| Staphylococcus aureus | A | - | - | - |
| | B | - | - | - |
| | C | - | - | - |
| | D | - | - | - |
| | Control* | + | + | - |
| Streptococcus pyogenes | A | - | - | - |
| | B | - | - | - |
| | C | - | - | - |
| | D | - | - | - |
| | Control* | + | + | - |
| Candida albicans | A | - | - | - |
| | B | - | - | - |
| | C | - | - | - |
| | D | - | - | - |
| | Control* | + | + | - |

\* Sterile water was used for control.

The results shown in Table 12 reveal that the p-oxybenzoic acid esters exhibit a potent bacteriocidal effect on Pseudomonas aeruginosa, Staphylococcus aureus, Streptococcus pyogenes and Candida albicans.

Example 7

Bacteriocidal effect on Escherichia coli and methicillin-resistant Staphylococcus aureus

1) Preparation of composition:

Solutions of p-oxybenzoic acid esters having compositions given in Table 13 below were prepared.

Table 13

| Composition | | | | |
|---|---|---|---|---|
| Composition | Active Ingredient | | Carrier | |
| | Compound | Amount (vol%) | Compound | Amount (vol%) |
| A | Methyl p-oxybenzoate | 0.5 | Ethanol Water | 70 30 |
| B | Ethyl p-oxybenzoate | 0.5 | Ethanol Water | 70 30 |
| C | Propyl p-oxybenzoate | 0.5 | Ethanol Water | 70 30 |
| D | Butyl p-oxybenzoate | 0.05 | Propylene glycol | 99.95 |
| E | Butyl p-oxybenzoate | 0.05 | Propylene glycol | 99.95 |
| F | Butyl p-oxybenzoate | 0.05 | Propylene glycol Ethanol | 30 70 |

2) Method:

Escherichia coli IFO 3301 and methicillin-resistant Staphylococcus aureus NS 462 (MRSA) (strain deposited in Japanese Food Composition Center Foundation) were used for the test. The test strains were subcultured in sub-culture medium (0.2% meat extract-supplemented bouillon medium, Eiken Kagaku K.K.) for up to 3 to 4 generations, and then transferred to the same medium followed by incubation at 35°C for 24 hours. The viral solution was thus obtained.

1 ml of each of the cell suspensions was added to and mixed with 10 ml of each of Compositions A through F shown in Table 13 above, the mixture was allowed to act at 20°C for a definite period of time. One platinum loop was taken from a test tube at 1 and 5 minutes in the case of Compositions A to C and E and in the case of Compositions D and F after 30 and 60 minutes, and transferred to a post-incubation medium SCDLP (Nippon Pharmaceutical Co., Ltd.). After incubation at 35°C for 2 hours, the test strains were observed to see whether they grew or not, to determine whether the bacteria survived or died.

For comparison, sterilized water was tested in a similar manner.

3) Results:

The results are shown in Table 14, wherein symbol + denotes that the bacteria were not dead and symbol - denotes that the bacteria were dead.

Table 14

| Test Results | | | | | | |
|---|---|---|---|---|---|---|
| Bacteria | Composi-tion | Survival or Death of Bacteria | | | | |
| | | 1′ | 5′ | 30′ | 60′ | |
| Escherichia coli | A | - | - | NT | NT | |
| | B | + | - | NT | NT | |
| | C | - | - | NT | NT | |
| | D | NT | NT | - | - | |
| | E | + | - | NT | NT | |
| | F | NT | NT | - | - | |
| | Control* | + | + | + | + | |
| MRSA | A | - | - | NT | NT | |
| | B | + | - | NT | NT | |
| | C | - | - | NT | NT | |
| | D | NT | NT | - | - | |
| | E | + | - | NT | NT | |
| | F | NT | NT | - | - | |
| | Control* | + | + | + | + | |
| NT: not tested | | | | | | |

\* Sterilized water was used for control.

As demonstrated by the foregoing data, the p-oxybenzoic acid esters are safe and exhibit a potent bacteriocidal, antibacterial and bacteriostatic activity against bacteria and viruses which generally can only be destroyed or prevented with difficulty.

**Claims**

1. A bacteriocidal, antibacterial or bacteriostatic composition comprising as an active ingredient a p-oxybenzoic acid ester, which exhibits an bacteriocidal, antibacterial or bacteriostatic activity against cariogenic Streptococcus mutans, Helicobacter pylori, Mycobacterium tuberculosis, influenza virus, HIV, herpes simplex, Pseudomonas aeruginosa, Staphylococcus aureus, Streptococcus pyogenes, Candida albicans, Escherichia coli or methicillin-resistant Staphylococcus aureus.

2. A bacteriocidal, antibacterial and bacteriostatic composition according to claim 1, for the treatment and/or prevention of diseases caused by infection with cariogenic Streptococcus mutans, Helicobacter pylori, Mycobacterium tuberculosis, influenza virus, HIV, herpes simplex, Pseudomonas aeruginosa, Staphylococcus aureus, Streptococcus pyogenes, Candida albicans, Escherichia coli or methicillin-resistant Staphylococcus aureus.

3. A bacteriocidal, antibacterial and bacteriostatic composition according to claim 2, which is in the form of liquid, paste or jelly.

4. A bacteriocidal, antibacterial and bacteriostatic composition according to any one of claims 1 to 3, which further contains a solvent.

5. Use of p-oxybenzoic acid ester in the preparation of a medicament for the treatment and/or prevention of diseases caused by infection with cariogenic Streptococcus mutans, Helicobacter pylori, Mycobacterium tuberculosis, influenza virus, HIV, herpes simplex, Pseudomonas aeruginosa, Staphylococcus aureus, Streptococcus pyogenes, Canida albicans, Escherichia coli or methicillin-resistant Staphylococcus aureus.

European Patent
Office

**EUROPEAN SEARCH REPORT**

Application Number

EP 97 30 0734

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | PATENT ABSTRACTS OF JAPAN<br>vol. 96, no. 02, 29 February 1996<br>& JP 07 252105 A  (IWAO HISHIDA), 30 October 1995,<br>* abstract *<br>--- | 1-5 | A01N37/40<br>A61K31/235 |
| X | T.R. AALTO ET AL.:  "p-Hydroxybenzoic Acid Esters as Preservatives"<br>JOURNAL OF THE AMERICAN PHARMACEUTICAL ASSOCIATION, SCIENTIFIC EDITION,<br>vol. 42, no. 8, August 1953, WASHINGTON, US,<br>pages 449-57, XP002041666<br>* the whole document *<br>--- | 1-5 | |
| X | DATABASE  CHEMABS<br>CHEMICAL ABSTRACTS SERVICE, COLUMBUS, OHIO, US<br>91:151926, November 1979<br>NISHIDA, HIDEKAZU: "Inhibitory effects of photosensitizer 201 and butylparaben on Streptococcus mutans"<br>XP002041667<br>* abstract *<br>&<br>SHIKA IGAKU (1979), 42(1), 28-42 CODEN: SIGAAE;ISSN: 0371-0246,<br> 1979,<br>---<br>-/-- | 1-5 | TECHNICAL FIELDS SEARCHED     (Int.Cl.6)<br><br>A01N<br>A61K |

The present search report has been drawn up for all claims

| Place of search | Date of completion of the search | Examiner |
|---|---|---|
| THE HAGUE | 24 September 1997 | Muellners, W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

| | European Patent Office | EUROPEAN SEARCH REPORT | Application Number EP 97 30 0734 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE CHEMABS<br>CHEMICAL ABSTRACTS SERVICE, COLUMBUS, OHIO, US<br>85:187172, December 1976<br>WOZNIAK-PARNOWSKA, WANDA ET AL: "The rate of bactericidal action of pharmacopoeial preservatives"<br>XP002041668<br>* abstract *<br>&<br>ACTA POL. PHARM. (1976), 33(1), 107-13<br>CODEN: APPHAX,<br>1976, | 1-5 | |
| X | WO 93 15728 A (MED-GREEN, INC.)<br>* page 1, paragraph 1 *<br>* claims 9,10 * | 1-5 | |
| X | WO 92 18111 A (SMITH-KLINE BEECHAM PLC)<br>* page 2, line 34 - page 3, line 2 *<br>* example 1 *<br>* claims 1,4 * | 1-5 | TECHNICAL FIELDS SEARCHED (Int.Cl.6) |
| X | DE 39 43 562 A (SCHÜLKE & MAYR GMBH)<br>* page 3, line 3 - line 8 *<br>* page 3, line 27 - line 32; example 4D *<br>* page 7, line 40 - line 65 * | 1-5 | |

-/--

The present search report has been drawn up for all claims

| Place of search | Date of completion of the search | Examiner |
|---|---|---|
| THE HAGUE | 24 September 1997 | Muellners, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 0 819 380 A1

European Patent Office

**EUROPEAN SEARCH REPORT**

Application Number

EP 97 30 0734

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | DATABASE CHEMABS<br>CHEMICAL ABSTRACTS SERVICE, COLUMBUS, OHIO, US<br>126:4476, January 1997<br>MA, Y. ET AL: "Irreversible paraben inhibition of glycolysis by Streptococcus mutans GS-5"<br>XP002041669<br>* abstract * | 1-5 | |
| X | &<br>LETT. APPL. MICROBIOL. (1996), 23(5), 329-333 CODEN: LAMIE7;ISSN: 0266-8254, 1996,<br>--- | 1-5 | |
| P,X | DE 195 14 694 A (I. LEVI)<br>* the whole document *<br>--- | 1-5 | |
| P,X | DATABASE CHEMABS<br>CHEMICAL ABSTRACTS SERVICE, COLUMBUS, OHIO, US<br>An: 126:36915, January 1997<br>SEGAWA, HIROYUKI ET AL: "Stable nonirritant dentifrices containing p-hydroxybenzoates"<br>XP002041670<br>* abstract *<br>& JP 08 268 850 A (EARTH CHEMICAL CO.) 15 October 1996<br>----- | 1-5 | TECHNICAL FIELDS SEARCHED (Int.Cl.6) |

The present search report has been drawn up for all claims

| Place of search | Date of completion of the search | Examiner |
|---|---|---|
| THE HAGUE | 24 September 1997 | Muellners, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

21